# EUROPEAN PATENT APPLICATION

(11) **EP 2 514 785 A1**
(43) Date of publication of application: **24.10.2012**
(21) Application number: 12161594.2
(22) Date of filing: 27.03.2012
(51) Int. Cl.: C08J 5/00

(54) **Water gel and method for manufacturing the same, as well as, water retaining gel mat and method for manufacturing the same**

(30) Priority: 18.04.2011 JP 2011092347
(71) Applicant: Hirakawa Corporation, Chuo-ku Tokyo 103-0014 (JP)
(72) Inventor: Hirakawa, Shoichi, Chuo-ku, Tokyo 103-0014 (JP)
(74) Representative: Harrison, Michael Robert

(57) **Abstract**

[Problem to be Solved]
The present invention provides a water gel in the form of, for example, flakes with which the defects, such as malodor, discoloration, and hydration, associated with conventional water gels have been eliminated.

[Solution] The water gel 1 according to the present invention is provided by using, as raw materials, an alkaline solution, and a monomer, such as acrylic acid, acrylate, or acrylamide, which is added to the alkaline solution, the content of the monomer being approx. 10% to 20%; making a pH-value adjustment; adding a crosslinking agent, a photoprimer and a photosensitizer; and making a UV photo-curing treatment at a wavelength of 320 nm to 400 nm for photocrosslinking the monomer to produce a water gel in the form of, for example, flakes.

## Description

### [Technical Field]

The present invention relates to a water gel in the form of, for example, flakes with which the defects, such as malodor, discoloration, and hydration, associated with conventional water gels have been eliminated, and a method for manufacturing the same, as well as a water retaining gel mat and a method for manufacturing the same.

### [Background Art]

Conventionally, a number of methods of synthesizing high polymer materials have been available, and principal methods of them provide synthesis of linear polymers.

In recent years, using the photocrosslinking method for synthesizing of high polymer polymers having a special structure and performance has been attracting people's interest.

Photopolymerization is one of the major means for synthesizing high polymer materials, offering advantages of saving energy consumption, freedom from contamination, rapid reaction, convenient operation, and the like, and with the advent of a light source providing more uniform performance, and of relatively low price, doubling the interest of people.

A number of researches on synthesizing linear high polymer materials having a photosensitivity, such as printing materials, films, UV paints, adhesives, printing inks, and novel materials have been made. However, few reports about synthesis of high polymer water gel materials by photocrosslinking have been published.

Water gel is a crosslinked networked elastic macromolecule containing a large amount of water, and has a good biological compatibility, being already widely used in the field of biotechnology, medical science and the like.

In recent years, the water gel has exhibited good prospects as a novel material especially in the field of biological material and biotechnology, attracting people's attention with the water gel providing a noteworthy research theme in the field of functional high polymer.

Next, the water retaining gel mat, which is used in such a place as a bed room or living room in home, (for example, the water retaining gel mat for use as a bed article) will be discussed.

The water retaining gel for use in such an application absorbs water vapor in the air under a high humidity condition, while losing its own water under a low humidity condition, thus it is capable of keeping equilibrium between the quantity of water absorbed and that of water lost over the long term in the natural environment. Therefore, the weight of the water retaining gel itself can be maintained within a relatively narrow range.

At present, almost all water retaining gels available inside and outside Japan use water as the dispersion medium, and if used over a long period of time, a large amount of water will be lost.

Generally, a water retaining gel is formed by introducing hydrophobic radicals into a water soluble high polymer having a crosslinked structure. The crosslinked polymer which is expandable when encountered with water is a high polymer network system, having a flexible property, and it can absorb a large amount of water while keeping a definite shape, but on the other hand it can lose a large amount of water.

Either a water soluble high polymer or a hydrophylic high polymer can form a water gel if subjected to a definite chemical or physical crosslinking. Such high polymers can be divided into two broad general categories: naturally derived and synthetic. Naturally derived hydrophylic high polymers include polysaccharides (such as starch, cellulose, alginic acid, hyaluronic acid, and chitosan) and polypeptides (such as collagen, poly-L-lycine, and poly-L-glutamic acid).

Synthetic hydrophylic high polymers include acrylic acid and a derivative thereof (such as polyacrylic acid, polymethacrylic acid, polyacrylamide, or poly-N-polyacrylamide).

The water retaining gel has been already used as a high water-absorption and high water-retaining material in various fields. Examples of application include a measure against drought in drought areas, an agricultural film, an anti-condensation agent in architecture, a humidity adjusting agent, a water stop adjusting agent in the petrochemical industry, a dewatering of crude oil and product oil, a dust preventing agent in the mining industry, a freshness keeping agent and thickening agent for foodstuffs, and a drug carrier in medical care.

However, conventional water retaining gels present a problem of poor water retaining capacity, resulting in the water in the water retaining gel being lost, thus it has been difficult to use the water retaining gel mat employing such a water retaining gel as a mat article in such a place as a bed room or living room in home, and in such applications as massage and acupuncture, moistening, and medical treatment.

As disclosed in the patent document 1, the applicant of the present patent application has proposed the inventions relating to a gel mat including a hermetically sealed bag which is constituted by two layers of a film material, the bag being packed with gel flakes, and a method for manufacturing a gel mat, comprising the steps of cutting a film material to the outside dimensions of the gel mat and on the material conditions thereof; opposing two film materials having a plastic layer to each other for heating the plastic layer to fuse the surfaces of the two film materials for producing a bag from the opposed two film materials that is hermetically sealed at the periphery except for a portion to be used as an injection port; intermixing water, a monomer, a crosslinking agent, and an initiator to give a monomer solution, pouring the monomer solution into the bag from the injection port, and then closing the injection port; placing the bag, into which the monomer solution has been poured, flat on a heater for heating it to thoroughly polymerize the monomer solution in the bag for generating a flake polymer, i.e., a gel; and inspecting the processing quality, and packaging an inspection-passed product for storing it in a warehouse.

### [Patent Document]

[Patent document 1] Japanese Patent Laid-Open Publication No. 2009-219875

### [Disclosure of the Invention]

### [Problems to be Solved by the Invention]

Being grounded on the above-described prior art and the art disclosed in the patent document 1, the present invention is intended to provide a novel water gel in the form of, for example, flakes with which the defects, such as malodor, discoloration, and hydration, associated with conventional water gels have been eliminated, and a method for manufacturing the same, as well as a novel water retaining gel mat which can substantially restore the original weight thereof, absorbing or losing water, provided that it is left under the normal temperature and humidity conditions for a sufficient period of time.

### [Means for Solving the Problems]

The water gel according to the present invention provides, as a most important feature thereof, a water gel comprising, as raw materials, an alkaline solution and a monomer, such as acrylic acid, acrylate, or acrylamide, the monomer being added to the alkaline solution, the water gel being formed in the form of, for example, flakes by photocrosslinking in a UV photo-curing treatment.

### [Advantages of the Invention]

According to the invention as stated in claim 1 to claim 3, there is provided a water gel comprising, as raw materials, an alkaline solution and a monomer, such as acrylic acid, acrylate, or acrylamide, the monomer being added to the alkaline solution, the water gel being formed by photocrosslinking in a UV photo-curing treatment, being instantaneously cured, whereby a novel water gel can actually be provided which is capable of maintaining a high elasticity over a long period of time, offering excellent physical features, such as freedom from crease.
According to the invention as stated in claim 4, there can actually be provided a novel water gel which is capable of exerting the effects of the invention as stated in claim 1 to claim 3, the water gel being formed in the form of flakes.

According to the invention as stated in claim 5 to claim 7, there is provided a method for manufacturing of a water gel, comprising the steps of:
dissolving a base in an aqueous solvent to prepare a mixed solution, and agitating the mixed solution to make an alkaline solution;
adding a monomer, such as acrylic acid, acrylate, or acrylamide, to the alkaline solution to prepare a mixed solution, and agitating the mixed solution to produce a mother liquid;
cooling the mother liquid to below a prescribed temperature;
adjusting the pH value of the mother liquid to a prescribed value;
adding a crosslinking agent, a photoprimer, and a photosensitizer to the mother liquid; and
pouring the mother liquid into a container, and making a UV photo-curing treatment for photocrosslinking the monomer to form a water gel, whereby a novel method for manufacturing of a water gel can actually be provided which is capable of manufacturing a novel water gel having excellent physical features as described above while the production speed being substantially improved.
According to the invention as stated in claim 8, there can actually be provided a novel water gel which can exert the effects of the invention as stated in claim 5 to claim 7, the water gel being formed in the form of flakes.

According to the invention as stated in claim 9 to claim 11, there can actually be provided a novel water retaining gel mat which, if placed under any temperature and humidity conditions, can substantially restore the original weight thereof, absorbing or losing water, provided that it is left under the normal temperature and humidity conditions for a sufficient period of time, whereby it can actually provide a novel water retaining gel mat which is suitable for use as, for example, a bed article in the bed room.

According to the invention as stated in claim 12 to claim 14, there is provided a method for manufacturing of a water retaining gel mat, comprising the steps of:
dissolving a base, such as sodium hydroxide, in an aqueous solvent to prepare a mixed solution; agitating the mixed solution to make an alkaline solution; and cooling thereof;
upon the temperature of the alkaline solution being lowered to around a prescribed temperature, adding glycerin or the like, the glycerin or the like being a water retaining factor, to the alkaline solution to prepare a mixed solution, and agitating the mixed solution;
adding acrylic acid, the acrylic acid being a monomer, to the solution to prepare a mixed solution; agitating the mixed solution; and cooling thereof to below a prescribed temperature for producing a mother liquid;
adjusting the pH value of the mother liquid to a prescribed value;
adding a crosslinking agent to the mother liquid to prepare a mixed solution, and agitating the mixed solution;
adding a primer and an initiator to the mother liquid to prepare a final mixed solution for water retaining gel formation;
preparing a bag body in the form of a mat, including an enclosing member constituted by a structure superposing a fabric cloth and a plastic layer one upon another, the peripheral portion being hermetically sealed except for an injection port for injecting the final mixed solution for water retaining gel formation;
injecting the final mixed solution for water retaining gel formation into the bag body; removing the bubbles; and sealing the injection port of the bag body by heat-welding; and
heat-pressing the bag body, the final mixed solution for water retaining gel formation being hermetically sealed therein, for causing the final mixed solution for water retaining gel formation to polymerize to form a water retaining gel in the form of flakes in the bag body for use as a water retaining gel mat,
whereby a novel method for manufacturing of a water retaining gel mat can actually be provided which is capable of manufacturing a novel water retaining gel mat exerting the excellent effects as described above.
According to the invention as stated in claim 15, there can actually be provided a novel water retaining gel mat which can exert the effects of the invention as stated in claim 12 to claim 14, the water gel being formed in the form of flakes.

### [Brief Description of the Drawings]

FIG. 1 is a schematic perspective view of a water gel according to an Embodiment 1 of the present invention;
FIG. 2 is a flowchart illustrating a manufacturing process for a water gel according to the Embodiment 1 of the present invention;
FIG. 3 is a schematic sectional view of a water retaining gel mat according to the Embodiment 2 of the present invention; and
FIG. 4 is a flowchart illustrating a manufacturing process for a water gel according to the Embodiment 2 of the present invention.

### [Mode for Carrying Out the Invention]

The present invention has achieved the purpose of providing a novel water gel with which the defects, such as malodor, discoloration, and hydration, associated with conventional water gels have been eliminated,
by using, as raw materials, an alkaline solution, and a monomer, such as acrylic acid, acrylate, or acrylamide, which is added to the alkaline solution, the content of the monomer being approx. 10% to 20%; making a pH-value adjustment; adding a crosslinking agent, a photoprimer and a photosensitizer; and making a UV photo-curing treatment at a wavelength of 320 nm to 400 nm for photocrosslinking the monomer to produce a water gel in the form of, for example, flakes.

### [Embodiments]

Hereinbelow, a water gel according to an Embodiment 1 of the present invention and a method for manufacturing the same, as well as a water retaining gel mat and a method for manufacturing the same will be explained in detail.

### (Embodiment 1)

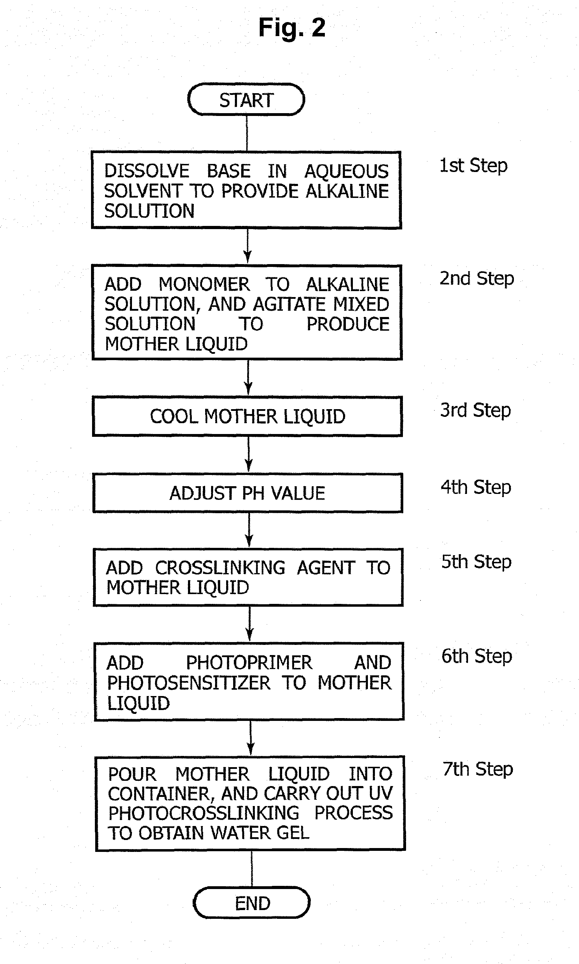

A water gel 1 according to an Embodiment 1 of the present invention and a method for manufacturing the same will be explained. The water gel 1 according to the present embodiment 1 is manufactured through the respective manufacturing steps as illustrated in FIG. 2.

### (1st step)

A base, such as sodium hydroxide, is dissolved in an aqueous solvent to prepare a mixed solution, and the mixed solution is thoroughly agitated to provide an alkaline solution, before being cooled.

### (2nd step)

When the temperature of the alkaline solution is lowered to approx. 25°C, a monomer, such as acrylic acid, acrylate, or acrylamide is added to the alkaline solution to prepare a mixed solution, and the mixed solution is agitated until it is lucid and transparent. The solution obtained herein is hereinafter abbreviated as the "mother liquid". The content of the monomer in the mother liquid is approximately 10% to 20%.

### (3rd step)

The mother liquid obtained at the 2nd step is cooled to below 25°C.

### (4th step)

The pH value of the mother liquid is measured, and an acid or base is added to the mother liquid for adjusting the pH measurement to 6.5 to 7.0.

### (5th step)

To the mother liquid passed through the 4th step, a polyvinyl alcohol solution, which is a crosslinking agent, is added to prepare a mixed solution, and the mixed solution is agitated for thorough dissolution. The content of the polyvinyl alcohol solution shall be approximately 1% to 5%.

### (6th step)

To the solution obtained at the 5th step, a photoprimer, such as a benzoin derivative, and a photosensitizer, such as a benzophenon, are added. The content of the photoprimer and the photosensitizer shall be approximately 1% to 3%, respectively.

### (7th step)

The mother liquid passed through the 6th step is poured into a fixed container, and subjected to a UV (ultraviolet rays) photocuring process as described below for photocrosslinking to form the water gel 1 in the form of, for example, flakes or mat flakes. In this case, the size of the water gel 1 formed in the form of flakes or mat flakes is not critical.

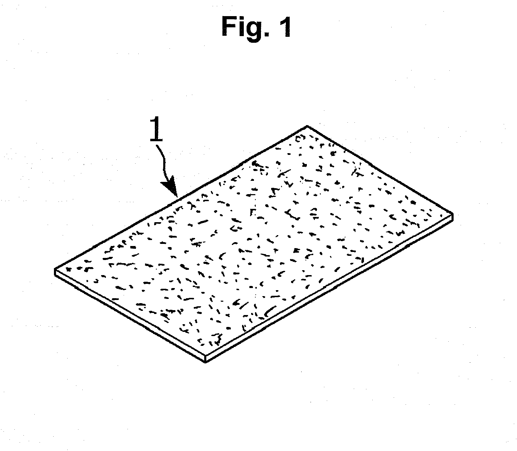

Through the above-described series of steps, manufacturing of the water gel 1 according to the present embodiment 1 as shown in, for example, FIG. 1 that has photo-initiation properties is completed.

The above-mentioned UV curing process is as follows:

A UV photocuring apparatus including a UV light tube irradiation apparatus, a cooling apparatus, a power supply, and a conveyor is used, and on the belt conveyor of the UV photocuring apparatus, the above-described container into which the mother liquid has been poured at the 7th step is loaded.

Next, after confirming that the UV photocuring apparatus is connected to the power supply, the cooling water valve and the air valve of the cooling apparatus are opened.

Next, operation of the belt conveyor is started, with the heat radiation fan being run.

Next, the power switch for the UV photocuring apparatus is turned on; and the UV light tube is lighted (for approximately 30 min) to heat the mother liquid in the container for UV photocrosslinking to form the water gel 1 in the form of, for example, flakes or mat flakes.

In this case, before starting the operation, it must be checked to make sure that the cooling water valve is opened; the belt conveyor has no faults; the voltmeter and the ammeter give a reading in the proper range, respectively; and the other necessary conditions are satisfied.
In case where the above-described UV photocuring apparatus is used to produce the water gel 1 in large quantity, the following volume-production steps are taken.

### (a) Light energy check

The probe of the light energy meter is inserted into just under the UV light tube, and this state is kept for 30 sec for observing and recording the reading of the light energy meter. In this case, measurement is made continuously in nine places for recording the uniformity of the amount of energy.

It is necessary that the average quantity of heat for the UV light tube be 800 to 1000 mJ, and if this requirement is not met, the UV light tube is not suited for carrying out volume production.

### (b) Adjustment of line speed

The line speed of the belt conveyor is adjusted to 0.5 to 2 m/min.

(c) In order to stop the operation, it must be checked to make sure that all the products on the belt conveyor have been fed out; the power switch for the UV light tube and that for the belt conveyor are turned off; the power switch for the fan is turned off in 30 min after the completion of the volume production; and the other necessary conditions are satisfied.

### (d) Other precautions

Other precautions include that, in order to assure the maximum initiation efficiency, the wavelength for the UV rays of the UV light tube must be between 320 nm and 400 nm; on the belt conveyor, the product must be kept placed flat; after turning off the UV light tube, it must not be immediately turned on again, and a time period of at least 30 min before turning it on again; the fan must not be stopped and the cooling water valve must not be closed immediately after the UV light tube being turned off; the UV light tube must be replaced with a new one at an appropriate time, because the service life of the UV light tube is approx. 800 to 1000 hours.

As described above, the water gel 1 according to the present embodiment 1 is manufactured by adding a monomer to an alkaline solution to produce a mother liquid; adjusting the pH value of this mother liquid to be 6.5 to 7.0; further adding a crosslinking agent, a photoprimer, and a photosensitizer to the mother liquid before being placed in a container; and using a UV photocuring apparatus to make UV photocrosslinking of the mother liquid.

According to the present embodiment 1, there can be provided a water gel 1 in the form of, for example, flakes or mat flakes that eliminates the defects, such as malodor, discoloration, and hydration, associated with water gels synthesized by the prior art.

Particularly, the UV photocuring process is a treatment requiring basically no solvent, thus a water gel 1 in the form of, for example, flakes that is green and free from malodor can be provided.

Further, according to the present embodiment 1, a water gel is formed by instantaneous curing in the UV photocuring process, thus a water gel 1 can actually be provided which is capable of maintaining a high elasticity over a long period of time, offering excellent physical features, such as freedom from crease.

Thereby, by using this water gel 1 in the form of flakes, a human body cooling apparatus containing the pertinent water gel can actually be provided which has a long service life, and can be widely used in such a place as a bed room or living room in home, and in such various applications as massage and acupuncture, moistening, and medical treatment.

Further, according to the present embodiment 1, there can actually be provided a method for manufacturing a water gel with which, through a series of steps, i.e., the 1st to 6th steps, and the 7th step, where a novel UV photocuring process using a UV photocuring apparatus provides photocrosslinking, the water gel 1 offering excellent physical properties as described above can be manufactured, and since the UV photocuring process features instantaneous curing, the production speed can be substantially improved.

Further, according to the present embodiment 1, there can actually be provided a method for manufacturing a water gel with which, by taking the volume production steps as described above, such as performing the light energy check and setting the proper line speed for the belt conveyor, the water gel 1 in the form of, for example, flakes or mat flakes that is capable of exerting the above-described function and effects can be efficiently produced in a large quantity.

### (Embodiment 2)

Hereinbelow, a water retaining gel mat 10 according to an Embodiment 2 of the present invention and a method for manufacturing the same will be explained in detail.

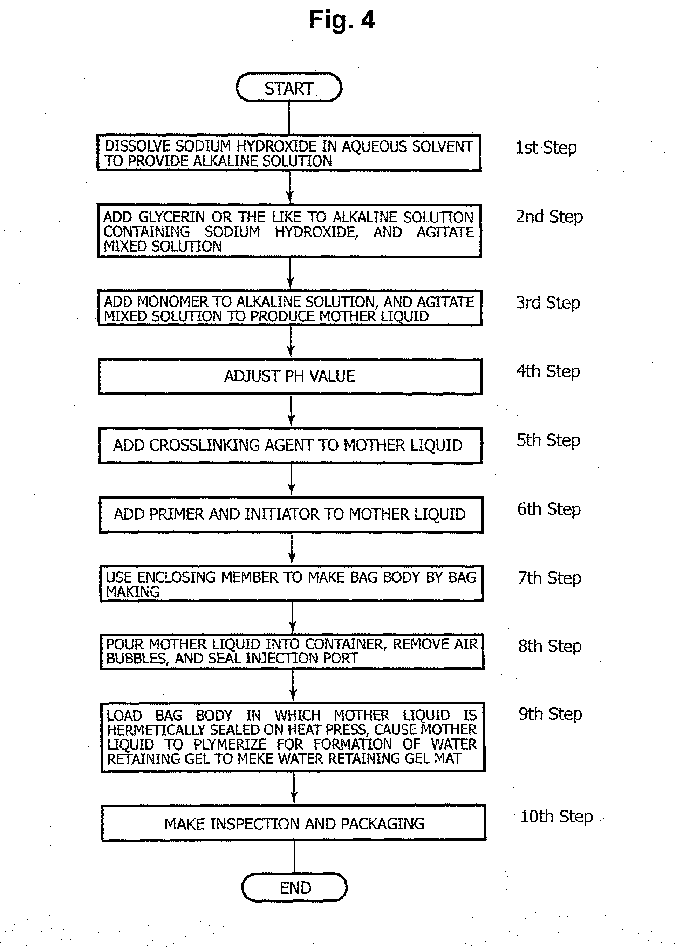

The water retaining gel mat 10 and a water retaining gel 21 according to the present Embodiment 2 are manufactured through the respective manufacturing steps as illustrated in FIG. 4.

### (1st step)

Sodium hydroxide is dissolved in an aqueous solvent to prepare a mixed solution, and the mixed solution is agitated for thorough dissolution before being cooled. The dosage of water shall be 10% to 30% of the total mass of the entire reaction system, and the dosage of sodium hydroxide shall be 5% to 20% of the total mass of the entire reaction system.

### (2nd step)

When the temperature of the sodium hydroxide solution is lowered to approx. 25°C, glycerin, ethyl cellulose, or polyacrylamide, which is a water retaining factor, is added to the alkaline solution to prepare a mixed solution, and the mixed solution is agitated until it is lucid and transparent.

The dosage of glycerin shall be 10% to 30% of the total mass of the entire reaction system, the dosage of ethyl cellulose shall be 10% to 15% of the total mass of the entire reaction system, and the dosage of polyacrylamide shall be 5% to 8% of the total mass of the entire reaction system.

### (3rd step)

Acrylic acid, which is a monomer, is added to the solution obtained at the 2nd step to prepare a mixed solution, and the mixed solution is agitated to be uniform before being cooled to below 25°C. The liquid obtained herein is hereinafter abbreviated as the "mother liquid".

### (4th step)

The pH value of the mother liquid is measured, and an acid or base is added to the mother liquid for adjusting the pH measurement to 6.5 to 6.7.

### (5th step)

To the mother liquid passed through the 4th step, benzoyl peroxide, N, N-methylene-bis acrylamide, or azobisiso butyronitrile, which is a crosslinking agent, is added to prepare a mixed solution, and the mixed solution is agitated for thorough dissolution. The dosage of each of them shall be 0.1% to 0.3% of the total mass of the entire reaction system.

### (6th step)

To the solution obtained at the 5th step, potassium persulfate, which is a primer, and a hydrogen peroxide solution, which is an initiator, are added. The dosage of potassium persulfate shall be 0.01% to 0.1% of the total mass of the entire reaction system, and the dosage of the hydrogen peroxide solution shall be 0.01% to 0.1% of the total mass of the entire reaction system.

### (7th step)

A) Preparation of enclosing member: In this step, the material for an enclosing member 11 is selected and cut to the outside dimensions of the water retaining gel mat 10 and on the material conditions thereof.

The enclosing member 11 used is comprised of two components each of which has a structure superposing a fabric cloth (or flocked fabric) 12 and a plastic layer 13 one upon another, the plastic layer 13 forming an internal layer.

B) Bag-making: Two enclosing members 11 are superposed one upon another with the plastic layer 13 forming the internal layer, the periphery of the opposed plastic layers 13 being partially heat-welded except for a portion to be used as an injection port (not shown in the figure), thus a bag body 15 having a hermetically sealed peripheral portion 14 being produced.

In the desired locations in this bag body 15 according to the size of the bag body 15, the plastic layers 13 opposed to each other may be partially heat-welded to provide one or plural partially welded portions 16 for restriction of migration of a water retaining gel 21 later described, and further provide a hole 17 in each of the partially welded portions 16.

If this is done, the finished water retaining gel mat 10 can be conveniently folded up at the locations of the partially welded portions 16, and the holes 17 can provide ventilation therethrough in service.

The geometry and size of the partially welded portions 16 may be determined according to the size of the bag body 15, and the partially welded portions 16 may be disposed in any patterns, such as linear and circular.

The fabric cloth 12 refers to a cotton cloth, synthetic fabric, cotton/synthetic fiber mixed spun fabric, or the like. The synthetic fabric to be used is nylon taffeta, or the like. The plastic layer 13 is formed of PVC, PE, PU, TPU or the like, the specified thickness of the plastic layer 13 being generally between 0.05 mm and 0.45 mm.

### (8th step)

C) Liquid injection: The liquid obtained at the 6th step is injected into the bag body 15; the bubbles are removed; and the injection port is heat welded for hermetically sealing the liquid in the bag body 15.

### (9th step)

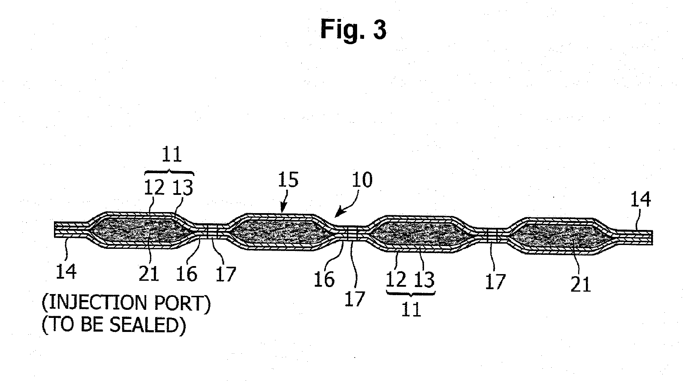

D) Heating for solidification: The bag body 15 in which the liquid is hermetically sealed is loaded horizontally on a hot press (not shown in the figure), and heat-pressed at a temperature of 70°C to 80°C for 10 to 20 min to cause a polymerization reaction to occur in the liquid in the bag body 15 for forming a water retaining gel 21 in the form of, for example, flakes or mat flakes in the bag body 15 to provide a water retaining gel mat 10 as shown in FIG. 3. In this case, the size of the water retaining gel 21 formed in the form of flakes or mat flakes is not critical.

The hot press pneumatically or hydraulically presses heat panels provided in two or three layers that are elevatably moved and the temperature of which can be adjusted between 30°C to 100°C as required. The bag body 15 is heated and pressed, being sandwiched between the heat panels.

### (10th step)

E) Inspection and packaging: The processing quality of the water retaining gel mat 10 is inspected, and the inspection-passed product is packaged to be stored in a warehouse. Now a series of steps is completed.

The step of bag-making as described above as the 7th step may not always be taken following the 6th step, in other words, the bag body 15 may be separately and previously manufactured such that the 8th to 10th steps can be taken following the 6th step.

Next, the normal water content restoring test conducted for the water retaining gel 21 formed at the 9th step as described above will be explained. In order to examine the normal water content restoring capacity of the water retaining gel 21, the following technique is adopted.

### (Technical conditions)

Testing equipment: Constant temperature and humidity test box at JIANGSU KUNSHAN QINGSHENG KEJIGUFENYOUXIANGONGSI
Dimensions: 90 cm x 140 cm
Material of enclosing member: Knitted fabric combined with TPU
Testing method: The weight of the sample is measured before being spread flat in the middle portion of the test box, and test is conducted under the respective sets of conditions as described below. The sample is left for 7 days in the environment of the constant temperature and humidity box (under the specified set of conditions) before being weighed.
After having been left for further 3 weeks in the normal temperature and humidity environment (at a temperature of 20 to 25°C, and a humidity of 50 to 70%), the sample is weighed. The test period shall be one month in total.

Table 1 gives the results of the test conducted with the constant temperature and humidity box being used under the four sets of conditions of a low temperature and a high humidity; a low temperature and a low humidity; a high temperature and a high humidity; and a high temperature and a low humidity.

**[Table 1]**

| Low temperature and high humidity (10°C and 90%) | | Low temperature and low humidity (10°C and 20%) | | High temperature and high humidity (70°C and 90%) | | High temperature and low humidity (70°C and 20%) | |
|---|---|---|---|---|---|---|---|
| Test item | Result | Test item | Result | Test item | Result | Test item | Result |
| Original weight | 5935 g | Original weight | 5919 g | Original weight | 5928 g | Original weight | 5915 g |
| Weight after 7 days | 5974 g | Weight after 7 days | 5628 g | Weight after 7 days | 6125 g | Weight after 7 days | 4890 g |
| Amount of absorbed water | 39 g | Amount of lost water | 291 g | Amount of absorbed water | 197 g | Amount of lost water | 1025 g |
| Percentage of absorbed water | 0.66% | Percentage of lost water | 4.91% | Percentage of absorbed water | 3.32% | Percentage of lost water | 17.33% |
| Weight after being left for 3 weeks at normal temp. and humidity | 5928 g | Weight after being left for 3 weeks at normal temp. and humidity | 5909 g | Weight after being left for 3 weeks at normal temp. and humidity | 5940 g | Weight after being left for 3 weeks at normal temp. and humidity | 5921 g |

The results of the test as given in Table 1 have revealed the following matters:
(a) Under the conditions of 10°C and humidity 90%, the sample absorbed water until the weight thereof reached 5974 g in 7 days, when the percentage of the absorbed water was 0.66%, and thereafter if left for 3 weeks under the normal temperature and humidity conditions inside the room, the sample lost water, the weight being gradually decreased to 5928 g, which is close to the original weight (5935 g).

(b) Under the conditions of 10°C and humidity 20%, the sample lost water until the weight thereof reached 5628 g in 7 days, when the percentage of the lost water was 4.91%, and thereafter if left for 3 weeks under the normal temperature and humidity conditions inside the room, the sample absorbed water, the weight being gradually increased to 5909 g, which is close to the original weight (5919 g).

(c) Under the conditions of 70°C and humidity 90%, the sample absorbed water until the weight thereof reached 6125 g in 7 days, when the percentage of the absorbed water was 3.32%, and thereafter if left for 3 weeks under the normal temperature and humidity conditions inside the room, the sample lost water, the weight being gradually decreased to 5940 g, which is close to the original weight (5928 g).

(d) Under the conditions of 70°C and humidity 20%, the sample lost water until the weight thereof reached 4890 g in 7 days, when the percentage of the lost water was 17.33%, and thereafter if left for 3 weeks under the normal temperature and humidity conditions inside the room, the sample lost water, the weight being gradually increased to 5921 g, which is close to the original weight (5915 g).

### (Conclusion)

As described above, it has been found that the water retaining gel 21 according to the present embodiment 2, if placed under any temperature and humidity conditions, can substantially restore the original weight thereof, absorbing or losing water, provided that it is left under the normal temperature and humidity conditions for a sufficient period of time (over 3 or longer weeks).

Thus, the water retaining gel mat 10 according to the present embodiment 2, if placed under any temperature and humidity conditions, can substantially restore the original weight thereof, absorbing or losing water, provided that it is left under the normal temperature and humidity conditions for a sufficient period of time, whereby it can actually provide a novel water retaining gel mat which is suitable for use as, for example, a bed article in the bed room.

In addition, with the method for manufacturing the water retaining gel mat 10 according to the present embodiment 2, the above-described series of steps allows obtaining a water retaining gel mat exerting the excellent effects as described above.

The water retaining gel mat 10 according to the present embodiment 2 can be formed so as to have a whole size of, for example, 90 cm wide x 180 cm long as a bed sheet size; 90 cm wide x 90 cm long as a half size; 40 cm wide x 30 cm long as a pillow size; or the like, to be used as a bed article, a mattress article, a pillow article, or the like, respectively, and the whole size is not limited to these.

### [Industrial Applicability]

The water gel according to the present invention can be applied in the field of biological material and biotechnology, and further, by packing it into a bag body, can be utilized as a human body cooling apparatus which can be widely used in such a place as a bed room or living room in home, and in such applications as massage and acupuncture, moistening, and medical treatment.
In addition, the water retaining gel mat according to the present invention is widely applicable as a mat article in such a place as a bed room or living room in home, and in such applications as massage and acupuncture, moistening, and medical treatment.

### [Description of Symbols]

- 1:: Water gel
- 10:: Water retaining gel mat
- 11:: Enclosing member
- 12:: Fabric cloth
- 13:: Plastic layer
- 14:: Peripheral portion
- 15:: Bag body
- 16:: Partially welded portion
- 17:: Hole
- 21:: Water retaining gel

## Claims

**1.**
A water gel comprising, as raw materials, an alkaline solution and a monomer, such as acrylic acid, acrylate, or acrylamide, the monomer being added to the alkaline solution,
the water gel being formed by photocrosslinking in a UV photo-curing treatment.

**2.**
A water gel comprising, as raw materials, an alkaline solution and a monomer, such as acrylic acid, acrylate, or acrylamide, the monomer being added to the alkaline solution, the content of the monomer being approx. 10% to 20%,
the water gel being formed by making a pH-value adjustment; adding a crosslinking agent, a photoprimer and a photosensitizer; and making a UV photo-curing treatment at a wavelength of 320 nm to 400 nm for photocrosslinking the monomer.

**3.**
A water gel comprising, as raw materials, an alkaline solution and a monomer, such as acrylic acid, acrylate, or acrylamide, the monomer being added to the alkaline solution, the content of the monomer being approx. 10% to 20%,
the water gel being formed by making a pH-value adjustment to a pH value of 6.5 to 7.0; adding a crosslinking agent constituted by a polyvinyl alcohol solution, the content of the crosslinking agent being approx. 1% to 5%; adding a photoprimer, such as a benzoin derivative, the content of the photoprimer being approx. 1% to 3%; adding a photosensitizer, such as benzophenon, the content of the photosensitizer being approx. 1% to 3%; and making a UV photo-curing treatment with UV light at a wavelength of 320 nm to 400 nm from a UV light tube placed above a belt conveyor for photocrosslinking the monomer.

**4.**
The water gel according to claim 1 to claim 3, wherein said water gel is formed in the form of flakes.

**5.**
A method for manufacturing of a water gel, comprising the steps of:
dissolving a base in an aqueous solvent to prepare a mixed solution, and agitating the mixed solution to make an alkaline solution;
adding a monomer, such as acrylic acid, acrylate, or acrylamide, to said alkaline solution to prepare a mixed solution, and agitating the mixed solution to produce a mother liquid;
cooling said mother liquid to below a prescribed temperature;
adjusting the pH value of said mother liquid to a prescribed value;
adding a crosslinking agent, a photoprimer, and a photosensitizer to said mother liquid; and
pouring said mother liquid into a container, and making a UV photo-curing treatment for photocrosslinking the monomer to form a water gel.

**6.**
A method for manufacturing of a water gel, comprising the steps of:
dissolving a base, such as sodium hydroxide, in an aqueous solvent to prepare a mixed solution; agitating the mixed solution to make an alkaline solution; and cooling thereof;
upon the temperature of said alkaline solution being lowered to approx. 25°C, adding a monomer, such as acrylic acid, acrylate, or acrylamide, to said alkaline solution to prepare a mixed solution, the content of the monomer being approx. 10% to 20%, and agitating the mixed solution to produce a mother liquid;
cooling said mother liquid to below 25°C;
adjusting the pH value of said mother liquid to 6.6 to 7.0;
adding a crosslinking agent constituted by a polyvinyl alcohol solution to said mother liquid to prepare a mixed solution, the content of the polyvinyl alcohol solution being approx. 1% to 5%, and agitating the mixed solution;
adding a photoprimer, such as a benzoin derivative, the content of the photoprimer being approx. 1% to 3%, and a photosensitizer, such as benzophenon, the content of the photosensitizer being approx. 1% to 3%; and
pouring said mother liquid into a container, and making a UV photo-curing treatment at a wavelength of 320 nm to 400 nm for photocrosslinking the monomer to form a water gel.

**7.**
A method for manufacturing of a water gel, comprising the steps of:
dissolving a base, such as sodium hydroxide, in an aqueous solvent to prepare a mixed solution; agitating the mixed solution to make an alkaline solution, and cooling thereof;
upon the temperature of said alkaline solution being lowered to approx. 25°C, adding a monomer, such as acrylic acid, acrylate, or acrylamide, to said alkaline solution to prepare a mixed solution, the content of the monomer being approx. 10% to 20%, and agitating the mixed solution to produce a mother liquid;
cooling said mother liquid to below 25°C;
adjusting the pH value of said mother liquid to 6.6 to 7.0;
adding a crosslinking agent constituted by a polyvinyl alcohol solution to said mother liquid to prepare a mixed solution, the content of the polyvinyl alcohol solution being approx. 1% to 5%, and agitating the mixed solution;
adding a photoprimer, such as a benzoin derivative, the content of the photoprimer being approx. 1% to 3%, and a photosensitizer, such as benzophenon, the content of the photosensitizer being approx. 1% to 3%; and
pouring said mother liquid into a container; loading the container on a belt conveyor having a line speed of 0.5 to 2 m/min; and making a UV photo-curing treatment for photocrosslinking the monomer to form a water gel under the conditions of the UV light from a UV light tube having a wavelength of 320 nm to 400 nm, and the average value of the quantities of heat from the UV light tube measured at a plurality of places being at 800 to 1000 mJ.

**8.**
The method for manufacturing of a water gel according to claim 5 to claim 7, wherein said water gel is formed in the form of flakes.

**9.**
A water retaining gel mat, comprising:
a bag body in the form of a mat, the peripheral portion being hermetically sealed except for an injection port, and the injection port being sealed after injection of a final mixed solution for water retaining gel formation therethrough; and
a water retaining gel formed in said bag body in the form of flakes by using, as raw materials, an alkaline solution, glycerin, said glycerin being a water retaining factor, and acrylic acid, said acrylic acid being a monomer, the raw materials being added to the alkaline solution; making a pH-value adjustment; adding a crosslinking agent, a primer and an initiator; and making pressing and heating the final mixed solution for water retaining gel formation injected into said bag body and hermetically sealed therein by sealing the injection port for causing a polymerization reaction of the monomer.

**10.**
A water retaining gel mat, comprising:
a bag body in the form of a mat, including an enclosing member constituted by a structure superposing a fabric cloth and a plastic layer one upon another, the peripheral portion being hermetically sealed except for an injection port, and the injection port being sealed after injection of final mixed solution for water retaining gel formation therethrough; and
a water retaining gel formed in said bag body in the form of flakes by using, as raw materials, an alkaline solution prepared by dissolving a base, such as sodium hydroxide, in an aqueous solvent to prepare a mixed solution, and agitating the mixed solution; glycerin, ethyl cellulose, or polyacrylamide, said glycerin, ethyl cellulose, or polyacrylamide being a water retaining factor, and acrylic acid, said acrylic acid being a monomer, the raw materials being added to the alkaline solution; making a pH-value adjustment; adding a crosslinking agent, a primer and an initiator to prepare a final mixed solution for water retaining gel formation; and making pressing and heating the final mixed solution for water retaining gel formation injected into said bag body and hermetically sealed therein by sealing the injection port for causing a polymerization reaction of the monomer.

**11.**
A water retaining gel mat, comprising:
a bag body in the form of a mat, including an enclosing member constituted by a structure superposing a fabric cloth and a plastic layer one upon another, the peripheral portion being hermetically sealed except for an injection port, and the injection port being sealed after injection of final mixed solution for water retaining gel formation therethrough; and
a water retaining gel formed in said bag body in the form of flakes by using, as raw materials, an alkaline solution prepared by dissolving a base, such as sodium hydroxide, at a dosage of 5% to 20% of the total mass of the entire reaction system in an aqueous solvent at a dosage of 10% to 30% of the total mass of the entire reaction system to prepare a mixed solution, and agitating the mixed solution; glycerin at a dosage of 10% to 30% of the total mass of the entire reaction system, ethyl cellulose at a dosage of 10% to 15% of the total mass of the entire reaction system, or polyacrylamide at a dosage of 5% to 8% of the total mass of the entire reaction system, said glycerin, ethyl cellulose, or polyacrylamide being a water retaining factor, and acrylic acid, said acrylic acid being a monomer, the raw materials being added to the alkaline solution; making a pH-value adjustment; adding benzoyl peroxide, N, N-methylene-bis acrylamide, or azobisiso butyronitrile, said benzoyl peroxide, N, N-methylene-bis acrylamide, or azobisiso butyronitrile being a crosslinking agent, each at a dosage of 0. 1% to 0.3% of the total mass of the entire reaction system; potassium persulfate, said potassium persulfate being a primer, at a dosage of 0.01% to 0. 1% of the total mass of the entire reaction system; and a hydrogen peroxide solution, said hydrogen peroxide solution being an initiator, at a dosage of 0.01 % to 0. 1% of the total mass of the entire reaction system to prepare a final mixed solution for water retaining gel formation; and making pressing and heating the final mixed solution for water retaining gel formation injected into said bag body and hermetically sealed therein by sealing the injection port for causing a polymerization reaction of the monomer.

**12.**
A method for manufacturing of a water retaining gel mat, comprising the steps of:
dissolving a base, such as sodium hydroxide, in an aqueous solvent to prepare a mixed solution; agitating the mixed solution to make an alkaline solution; and cooling thereof;
upon the temperature of said alkaline solution being lowered to around a prescribed temperature, adding glycerin or the like, said glycerin or the like being a water retaining factor, to the alkaline solution to prepare a mixed solution, and agitating the mixed solution;
adding acrylic acid, said acrylic acid being a monomer, to said solution to prepare a mixed solution, and agitating the mixed solution, and cooling thereof to below a prescribed temperature for producing a mother liquid;
adjusting the pH value of said mother liquid to a prescribed value;
adding a crosslinking agent to said mother liquid to prepare a mixed solution, and agitating the mixed solution;
adding a primer and an initiator to said mother liquid to prepare a final mixed solution for water retaining gel formation;
preparing a bag body in the form of a mat, including an enclosing member constituted by a structure superposing a fabric cloth and a plastic layer one upon another, the peripheral portion being hermetically sealed except for an injection port for injecting the final mixed solution for water retaining gel formation;
injecting the final mixed solution for water retaining gel formation into said bag body; removing the bubbles; and sealing the injection port of the bag body by heat-welding; and
heat-pressing the bag body, the final mixed solution for water retaining gel formation being hermetically sealed therein, for causing the final mixed solution for water retaining gel formation to polymerize to form a water retaining gel in the form of flakes in the bag body for use as a water retaining gel mat.

**13.**
A method for manufacturing of a water retaining gel mat, comprising the steps of:
dissolving a base, such as sodium hydroxide, in an aqueous solvent to prepare a mixed solution; agitating the mixed solution to make an alkaline solution; and cooling thereof;
upon the temperature of said alkaline solution being lowered to around 25°C, adding glycerin, ethyl cellulose, or polyacrylamide, said glycerin, ethyl cellulose, or polyacrylamide being a water retaining factor, to the alkaline solution to prepare a mixed solution, and agitating the mixed solution;
adding acrylic acid, said acrylic acid being a monomer, to said solution to prepare a mixed solution, and agitating the mixed solution, and cooling thereof to below 25°C for producing a mother liquid;
adjusting the pH value of said mother liquid to 6.5 to 7.0;
adding benzoyl peroxide, N, N-methylene-bis acrylamide, or azobisiso butyronitrile, said benzoyl peroxide, N, N-methylene-bis acrylamide, or azobisiso butyronitrile being a crosslinking agent, to said mother liquid to prepare a mixed solution, and agitating the mixed solution;
adding potassium persulfate, said potassium persulfate being a primer, and a hydrogen peroxide solution, said hydrogen peroxide solution being an initiator, to said mother liquid to prepare a final mixed solution for water retaining gel formation;
preparing a bag body in the form of a mat, including an enclosing member constituted by a structure superposing a fabric cloth and a plastic layer one upon another, the peripheral portion being hermetically sealed except for an injection port for injecting the final mixed solution for water retaining gel formation;
injecting the final mixed solution for water retaining gel formation into said bag body; removing the bubbles; and sealing the injection port of the bag body by heat-welding; and
heat-pressing the bag body, the final mixed solution for water retaining gel formation being hermetically sealed therein, for causing the final mixed solution for water retaining gel formation to polymerize to form a water retaining gel in the form of flakes in the bag body for use as a water retaining gel mat.

**14.**
A method for manufacturing of a water retaining gel mat, comprising the steps of:
dissolving a base, such as sodium hydroxide, at a dosage of 5% to 20% of the total mass of the entire reaction system in an aqueous solvent at a dosage of 10% to 30% of the total mass of the entire reaction system to prepare a mixed solution; agitating the mixed solution to make an alkaline solution; and cooling thereof;
upon the temperature of said alkaline solution being lowered to around 25°C, adding glycerin at a dosage of 10% to 30% of the total mass of the entire reaction system, ethyl cellulose at a dosage of 10% to 15% of the total mass of the entire reaction system, or polyacrylamide at a dosage of 5% to 8% of the total mass of the entire reaction system, said glycerin, ethyl cellulose, or polyacrylamide being a water retaining factor, to the alkaline solution to prepare a mixed solution, and agitating the mixed solution;
adding acrylic acid, said acrylic acid being a monomer, to said solution to prepare a mixed solution, and agitating the mixed solution, and cooling thereof to below 25°C for producing a mother liquid;
adjusting the pH value of said mother liquid to 6.5 to 7.0;
adding benzoyl peroxide, N, N-methylene-bis acrylamide, or azobisiso butyronitrile, said benzoyl peroxide, N, N-methylene-bis acrylamide, or azobisiso butyronitrile being a crosslinking agent, each at a dosage of 0. 1% to 0.3% of the total mass of the entire reaction system to said mother liquid to prepare a mixed solution, and agitating the mixed solution;
adding potassium persulfate, said potassium persulfate being a primer, at a dosage of 0.01 % to 0.1 % of the total mass of the entire reaction system and a hydrogen peroxide solution, said hydrogen peroxide solution being an initiator at a dosage of 0.01% to 0.1% of the total mass of the entire reaction system, to said mother liquid to prepare a final mixed solution for water retaining gel formation;
injecting the final mixed solution for water retaining gel formation into said bag body; removing the bubbles; and sealing the injection port of the bag body by heat-welding; and
heat-pressing the bag body at a temperature of 70°C to 80°C for 10 to 20 min, the final mixed solution for water retaining gel formation being hermetically sealed therein, for causing the final mixed solution for water retaining gel formation to polymerize to form a water retaining gel in the form of flakes in the bag body for use as a water retaining gel mat.

**15.**
The method for manufacturing of a water retaining gel mat according to claim 12 to claim 14, wherein said water retaining gel mat is formed in the form of flakes.
